(19) **Europäisches Patentamt**
**European Patent Office**
**Office européen des brevets**

(11) **EP 1 645 342 B1**

(12) **EUROPEAN PATENT SPECIFICATION**

(45) Date of publication and mention
of the grant of the patent:
**11.04.2012 Bulletin 2012/15**

(51) Int Cl.:
*B08B 3/12* *(2006.01)*     *B08B 3/02* *(2006.01)*
*B01J 19/10* *(2006.01)*     *H01L 21/02* *(2006.01)*
*H01L 21/67* *(2006.01)*     *A61L 2/02* *(2006.01)*

(21) Application number: **05447214.7**

(22) Date of filing: **20.09.2005**

(54) **Method and apparatus for controlled transient cavitation**

Verfahren zur kontrollierten transienten Kavitation

Procédé de cavitation transitoire contrôlée

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **21.09.2004 EP 04447204
21.09.2004 US 612087 P**

(43) Date of publication of application:
**12.04.2006 Bulletin 2006/15**

(73) Proprietors:
• **IMEC**
**3001 Leuven (BE)**
• **SAMSUNG ELECTRONICS CO., LTD.**
**Yongin-City,**
**Gyec (KR)**

(72) Inventors:
• **Holsteyns, Frank**
**3052 Blanden (BE)**
• **Lee, Kuntack**
**Suwon-City, Gyunggi-Do**
**Sout Korea 443-400 (KR)**

(74) Representative: **Bird, William Edward et al**
**Bird Goën & Co**
**Klein Dalenstraat 42A**
**3020 Winksele (BE)**

(56) References cited:
**EP-A- 0 860 866     DE-A- 4 305 660
US-A- 5 437 729     US-A- 5 681 396
US-A- 5 931 173     US-A- 5 961 895
US-A- 6 048 405**

• **SUSLICK K S ED - SCHNEIDER S C ET AL:
"Sonoluminescence and sonochemistry",
ULTRASONICS SYMPOSIUM, 1997.
PROCEEDINGS., 1997 IEEE TORONTO, ONT.,
CANADA 5-8 OCT. 1997, NEW YORK, NY, USA,
IEEE, US, vol. 1, 5 October 1997 (1997-10-05),
pages 523-532, XP010271279, DOI:
10.1109/ULTSYM.1997.663076 ISBN:
978-0-7803-4153-1**

**Description**

**Field of the invention**

The invention relates to a method for creating and controlling transient cavitation in a liquid. The method may be applied in a wide range of applications including cleaning, e.g. cleaning of medical apparatus, cleaning of flat substrates such as wafers in the semiconductor industry, etc.

It also relates to an apparatus suitable for creating and controlling transient cavitation in a liquid, especially to cleaning apparatus.

**Background of the invention**

Cavitation is generally known and defined as the activity of bubbles in a liquid. This bubble activity comprises bubble growth, pulsation or collapse. The pulsation of bubbles is known as stable cavitation, whereas the collapse of bubbles is known as transient cavitation. The latter can release high amounts of energy towards the surroundings via heat, shockwaves, etc.

Transient cavitation is applied in a large number of technical fields. In sonochemistry, bubbles collapsing in an ultrasonic field have a catalytic effect on chemical reactions. Also in medical applications cavitation is used, for example as contrast enhancer in ultrasound diagnostics. Probably the best-known application of cavitation is the removal of particles from a surface of a substrate.

A common problem in these various applications is how to control this transient cavitation phenomenon such that it performs in a controlled uniform way with respect to location and appearance of collapse.

For example, in cleaning technology today, a common problem is non-uniform removal of particles from the surface of a substrate. The particles have to be removed without damaging the substrate by heavy collapse and nevertheless leaving a uniform cleaned surface.

The particle removal mechanism was studied earlier either by gasifying with gasses like oxygen, nitrogen, argon, xenon, carbon dioxide, etc., or by degassing the liquid to get ultra pure water and then looking at the sonoluminescence (SL) signal generated by collapsing bubbles and the particle removal efficiency.

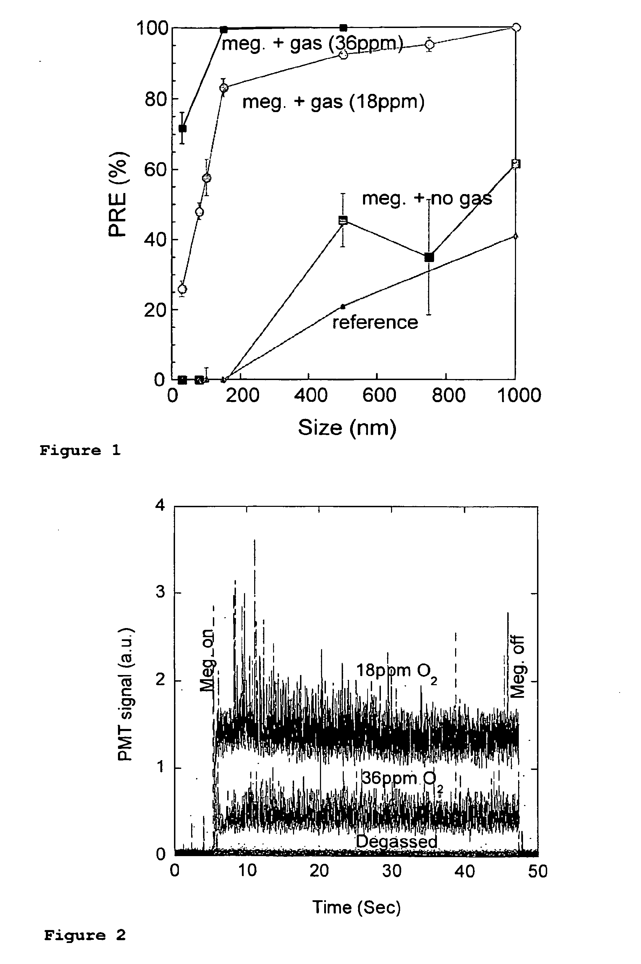

In figure 1 is shown that the presence of gas in the liquid is necessary to achieve high particle removal efficiency (PRE). In figure 2 the SL signal is plotted for gasified and degassed liquid. In gasified liquids an SL signal, generated by collapsing bubbles, can be detected contrary to degassed liquid. The combination of both figures pointed out that transient cavitation is on the basis of the particle removal mechanism.

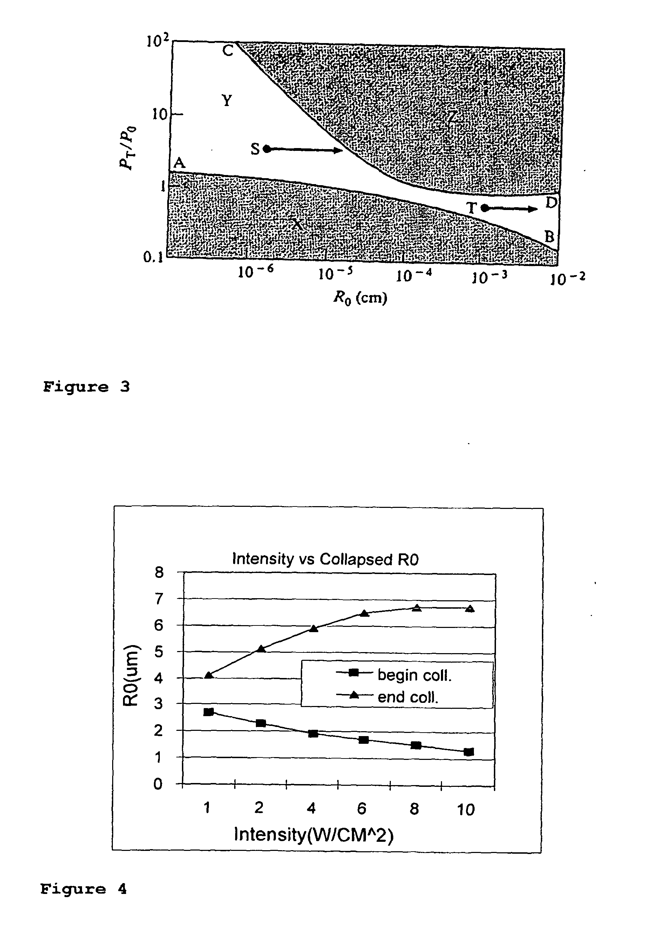

In figure 3, Neppiras (Acoustic Cavitation, Physics Reports 61, (1980), p.159-251) shows that dependent on the driving pressure ($P_T/P_O$) and the frequency of the acoustic field and dependent on the gas bubble radius ($R_O$), the gas bubble can grow (zone Y) and then collapse when entering area Z, or can dissolve again, if small enough to enter zone X.

In US6048405 Skrovan teaches a cleaning method for microelectronics industry introducing gas bubbles below the surface of the substrate such that the bubbles pass across the surface as they rise in the liquid and immersing the surface in a particular region through which megasonic energy is projected. However, Skrovan claims the particles are released from the surface by shockwaves from the megasonic field and not by shockwaves from transient cavitation. However, figure 1 proofs that the use of only megasonic energy is not efficient at all compared to gasified liquid in combination with megasonic energy. US-A-5931173 relates to monitoring cleaning effectiveness of cleaning systems and methods in which vibrations are applied to a volume of fluid in which an object to be cleaned is at least partially immersed.

In general, the state of a physical characteristic of the fluid during the application of the vibrations is monitored, thus providing a more direct and accurate measure of the effectiveness of the cleaning. According to an embodiment, the physical characteristic of the fluid may be the formation of bubbles on a surface that is immersed in the fluid to enable the size and/or frequency of formation of such surface bubbles to be determined. The surface bubble size and frequency of formation can be displayed by the system controller and/or used by the system controller to automatically control the cleaning process.

DE-A-4305660 describes a process for controlling the size distribution of gas or liquid bubbles in a liquid medium. The method comprises coupling an ultra sound field to the bubble-containing liquid medium and selecting the sound frequency and associated energy so that bubbles with diameters which can be determined at these frequencies and energies are set in resonance vibrations until they exceed the oscillation amplitude Ac necessary to break the bubbles up into smaller bubbles.

An advantage of the method according to DE-A-4305660 is that the maximum diameter of the bubbles, which after applying the ultra sound field are still present in the liquid, can be well controlled.

In "Sonoluminescence and Sonochemistry" Kenneth S. Suslick describes that oscillations of a gas bubble driven by an acoustic field are generally described by the Rayleigh-Plesset equation. This document discloses a method in accordance with the preamble of claim 1 and an apparatus in accordance with the preamble of claim 17, and describes the effect that a change of parameters of the acoustic field can have to the bubble size.

## Summary of the invention

In a first aspect the invention provides a method for creating transient cavitation in accordance with claim 1, comprising the steps of

- creating gas bubbles having a specific bubble size distribution in a liquid,
- creating an acoustic field and
- subjecting the liquid to the acoustic field, characterized in that the method furthermore comprises tuning one or both of the bubble size distribution or the characteristics of the acoustic field to each other in accordance with the Rayleigh-Plesset model so as to thereby control transient cavitation in the bubble size distribution.

The liquid can be any of, or any combination of, an aqueous solution, an organic, inorganic, polar or nonpolar solvent, a mixture of chemicals or any fluid which a gas can be dissolved or injected. The method can be used in any cleaning application, e.g. for medical equipment or for wafers in the semiconductor industry.

The characteristics of the acoustic field to be selected can be any of, or any combination of, the frequency, the intensity, the position of the transducer or transducers, or any parameter affecting the acoustic field.

In an embodiment of the invention, the specific bubble size distribution can be selected subsequent to and depending on the selection of the characteristics of the acoustic field. If the characteristics of the acoustic field are fixed or the selection thereof is limited, the range of bubble sizes can be adjusted.

In a further embodiment of the invention, the characteristics of the acoustic field can be selected subsequent to and depending on the specific bubble size distribution. If the specific bubble size distribution is fixed or the selection thereof is limited, the characteristics of the acoustic field can be adjusted.

In an embodiment of the invention, the step of creating gas bubbles having a specific bubble size distribution may comprise dissolving gas in the liquid by means of a gasification unit or any other means to dissolve gas in the liquid.

In an embodiment of the invention, the step of creating gas bubbles having a specific bubble size distribution may comprise injection of gas in the liquid by means of a bubbler system, a capillary, a nozzle, a membrane contactor or any means to inject gas bubbles into the liquid.

In an embodiment of the invention, the step of creating gas bubbles having a specific bubble size distribution may comprise applying a pressure drop, preferably a rapid pressure drop or may comprise applying one or multiple compression/decompression cycles.

In an embodiment of the invention, the step of creating gas bubbles having a specific bubble size distribution may comprise a raise in temperature or may comprise one or multiple cycles of heating/cooling the liquid.

In an embodiment of the invention, the step of creating gas bubbles having a specific bubble size distribution may comprise a step of subjecting the liquid to an additional acoustic field.

In an embodiment of the invention, the step of creating gas bubbles having a specific bubble size distribution sizes may comprise dissolving or injecting two or more different gasses into the liquid.

In an embodiment of the invention, the step of creating gas bubbles having a specific bubble size distribution may comprise adding a surfactant to the liquid.

In an embodiment of the invention, the specific bubble size distribution may be varied over time.

In an embodiment of the invention, the acoustic field may comprise more than one frequency.

An embodiment of the invention can further comprise the step of measuring the specific bubble size distribution.

An embodiment of the invention can further comprise the step of controlling the characteristics of the acoustic field subsequently to and dependent on the measurement of the specific bubble size distribution.

An embodiment of the invention can further comprise the step of controlling the bubble size distribution subsequently and dependent on the measurement of the range of bubble sizes.

In a second aspect, the invention provides an apparatus in accordance with claim 17, comprising

- means for creating gas bubbles having a specific bubble size distribution in a liquid,
- means for creating an acoustic field and
- means for subjecting the liquid to the acoustic field, characterized in that one or both of the means for creating the gas bubbles or the means for creating the acoustic field are adapted to tune the bubble size distribution and the characteristics of the acoustic field to each other in accordance with the Rayleigh-Plesset model to thereby control transient cavitation in the bubble size distribution.

In an apparatus according to the invention, the means for creating gas bubbles having a bubble size distribution may comprise a gasification unit or any means to dissolve gas in the liquid.

In an apparatus according to the invention, the means for creating gas bubbles having a bubble size distribution may comprise a valve, a nozzle, a membrane contactor or any means to inject gas bubbles in the liquid.

In an apparatus according to the invention, the means for creating gas bubbles having a bubble size distribution may comprise a heat exchanging system or any means for heating/cooling the liquid.

In an apparatus according to the invention, the means for creating gas bubbles having a bubble size distribution may comprise any means for generating a pressure drop or generating one or multiple compression/decompression cycles.

In an apparatus according to the invention, the means for creating gas bubbles having a bubble size distribution may comprise means to create an additional acoustic field and to subject the liquid to the additional acoustic field.

In an apparatus according to the invention, the means for creating gas bubbles having a bubble size distribution may comprise means for dissolving or injecting two or more different gasses into the liquid.

In an apparatus according to the invention, the means for creating gas bubbles having a bubble size distribution may comprise means for adding a surfactant to the liquid.

In an apparatus according to the invention, the means for creating an acoustic field may be capable of generating more than one frequency.

An apparatus according to the invention can further comprise a measurement unit for measuring bubble size distribution .

An apparatus according to the invention can further comprise a control unit for controlling the means for creating gas bubbles having a bubble size distribution and/or controlling the means for creating an acoustic field.

### Short description of the drawings

Figure 1 : PRE as a function of particle size and gas concentration in the liquid.

Figure 2 : SL signal as a function of time and gas concentration in the liquid.

Figure 3 : Behavior of a gas bubble in a liquid as a function of acoustic field characteristics and bubble size.

Figure 4 : Collapse as a function of bubble size and acoustic field characteristics.

Figure 5 : Maximum temperature and pressure during collapse as a function of frequency.

Figure 6 : Set-up with 2 sets of transducers, each set comprising a plurality of transducers, which can be used with the present invention.

Figure 7 : Process flow of a particular set-up which can be used with the present invention.

Figure 8 : Schematic of a spray nozzle.

Figure 9 : PRE for 1.8 MHz, 5 W/cm$^2$, 2.5 bar overpressure, 18 ppm oxygen.

Figure 10 : PRE for 1.8 MHz, 5 W/cm$^2$, 2.5 bar overpressure, 10 ppm oxygen.

Figure 11 : Amount of bubbles versus saturation level.

### Detailed description of the invention

The present invention relates to methods and apparatus for creating transient cavitation in a liquid. Such methods and apparatus can be used for cleaning purposes, e.g. in medical applications where sterilization is required, or in the semiconductor industry for cleaning wafers. The invention will be described in certain embodiments and with reference certain drawings, however these are for explanation purposes only.

Transient cavitation is a form of cavitation whereby the main activity of the bubbles in the liquid is bubble collapse. This collapse can release high amounts of energy towards the surroundings via heat, shockwaves, etc.

In order to control transient cavitation in a liquid and solve the problem of non-uniform performance of transient cavitation, a method in accordance with claim 17 is disclosed hereinafter to generate a specific bubble distribution and to bring these bubbles into a specific acoustic field.

Creating bubbles in the liquid can be done in different ways. First of all, addition of gas to the liquid is needed. Gases like nitrogen, oxygen, carbon dioxide, argon, helium, Xenon, etc. can be added to the liquid by a gasification unit, e.g. a membrane contactor. The ambient pressure of the liquid, the hydrostatic pressure of the liquid, the vapor pressure of the gas, the liquid flow, the liquid temperature and the contact area between gas and liquid are the main parameters to control the amount of gas dissolved in the liquid. Most of the gas will be present in the dissolved state. The higher the pressure of the liquid and the lower the temperature the more gas can be dissolved in the liquid. The amount and the saturation level of the gas dissolved will have an influence on the bubble formation.

Bubble formation out of the dissolved gas can be obtained by one or multiple compression and decompression cycles. This can be done by using a pressure-releasing valve to obtain a pressure drop in the tank after compression. Also a valve, an orifice or a membrane between two tank compartments of which one is at higher pressure than the second one can supply a pressure drop, thereby creating gas bubbles in the second compartment. Controlling the pressure difference and optionally the speed of cycles of compression/decompression is a way to control the amount and size of the gas bubbles. Another way to create bubbles out of dissolved gas is a raise in temperature by a heat exchanging system. The temperature shift is then the main parameter to determine the size of the bubbles.

Acoustic energy can also create bubbles in a liquid out of dissolved gas, because acoustic energy waves are pressure waves causing compression/decompression cycles.

Instead of forming bubbles out of dissolved gas, immediate injection of bubbles in the liquid can be applied. A bubbler system, a capillary, a nozzle, etc. can inject gas bubbles in the liquid. Also a membrane contactor with a dedicated pore size, separating the liquid from the supplied gas, can be used.

In general, the design of the hardware, the amount of gas present, the decompression rate, the temperature shift, the presence of surface-active agents (e.g. surfactants), etc. will determine the final size of the bubbles present in the liquid. The smallest bubbles will disappear again as dissolved gas and the bigger bubbles will grow. The critical size, determining either growth or disappearance, will be affected by the vapor pressure in the liquid, the ambient pressure of the liquid and the surface tension.

A few techniques to measure bubble sizes are available, for example light-scattering and sound dispersion.

In the light-scattering method, a laser beam is directed through a cell wherein a liquid containing gas bubbles is flowing in a continuous way perpendicular to the laser beam. A bubble crossing the laser beam causes light-scattering which is detected by a photodiode connected to an oscilloscope. The laser beam itself is blocked by a beam stopper after crossing the cell. The intensity of the light-scattering signal is a measure for the bubble size and is calibrated by means of latex sphere equivalent sizes. Optionally, this method is applicable in an acoustic field.

In the sound dispersion method, a transmitter is sending a signal towards a receiver in a fluid medium containing gas bubbles. The bubbles present in the fluid influence the attenuation and the group velocity of the signal. These two parameters can be calibrated as a measure for gas bubble size and number of bubbles. However, this method is not applicable in an acoustic field because of the influence of the acoustic waves on the signal.

The created gas bubbles or at least a part of it are subjected to an acoustic field with a typical acoustic pressure and a typical frequency, causing a particular bubble activity: growth, pulsation or collapse of bubbles. All bubbles are active but not all of them will collapse. Only bubbles with a particular distribution of bubble size will collapse, which is described by the Rayleigh-Plesset model, leading to transient cavitation. The following equation describes the adiabatic collapse of a gas bubble in a liquid:

$$\rho(R\ddot{R}+\frac{3}{2}\dot{R}^2)=P_g(R)-P_0-P_a(t)+\frac{R}{c}\frac{d}{dt}\left[P_g(R)-P_a(t)\right]-4\eta\frac{\dot{R}}{R}-\frac{2\sigma}{R}$$

wherein R is the bubble radius, $\rho$ is the liquid density, $P_g$ is the vapor pressure, $P_0$ is the ambient hydrostatic pressure, $P_a$ is the time dependent driving pressure, c is the speed of the acoustic waves in the liquid, $\eta$ is the liquid viscosity and $\sigma$ is the liquid surface tension. The driving pressure is dependent of the frequency and intensity of the acoustic field.

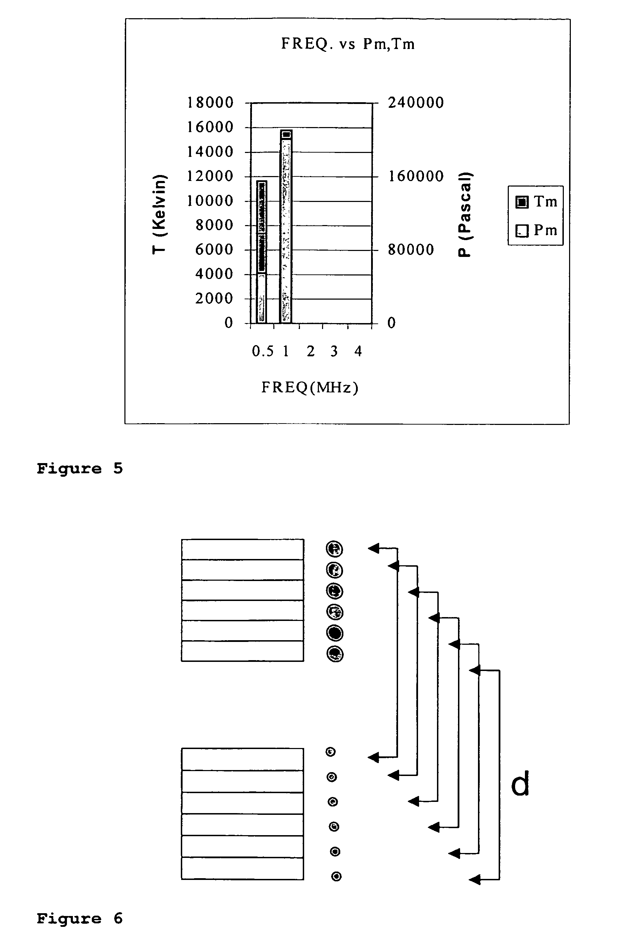

In case of particle removal, collapsing bubbles cause micro-streaming and shockwaves resulting in the creation of a drag force over the particles present on the surface. If these forces can overcome the Vanderwaals and Electrostatic forces these particles can be removed. In order to obtain a maximum quantity of micro-streaming and shockwaves in the liquid, as many gas bubbles as possible must collapse nearby the surface of the substrate. Theoretical calculations and experimental data show that only a specific range of bubble sizes collapse in a specific acoustic field. In figure 4 is shown that bubbles that are smaller than a minimum bubble size or bigger than a maximum bubble size do not collapse in an acoustic field with a particular intensity and a particular frequency, in this case 1 MHz. In figure 5 is shown that the maximum pressure Pm and the maximum temperature Tm during bubble collapse are dependent on the frequency of the acoustic field. When generating the appropriate bubble size in a particular acoustic field, transient cavitation can thus be controlled. In case of cleaning, the process conditions can be optimized in order to obtain a uniform particle removal and reduce the amount of damage on the surface by controlling violently collapsing bubbles.

In the general scope of this invention, the gas bubble size in the region where the acoustic field is provided and the specifications of the acoustic field have to be geared to each other in order to have a uniform transient cavitation or, in other words, bubble collapse, in that region. Thus, if on the one hand for particular reasons the useful range of gas bubble size is limited, the acoustic field has to be adjusted obtaining the desired cavitation. If on the other hand limitations on the specifications of the acoustic field have to be taken into account, the range and distribution of bubble size have to be adjusted.

A way to optimize the uniformity of transient cavitation in a tank is tuning the range of bubble sizes at the bottom of the tank by tuning the design of the valve, the orifice, the membrane contactor, etc. The bubbles will grow as they rise in the tank and are adapted to have the desired range of sizes when entering the acoustic field. Besides the initial size of the generated bubbles, the depth of generation, the depth of the tank and the position of the acoustic field can be varied in this case. The liquid flow speed and/or orientation is another parameter that can be varied and has a major influence on the size and distribution of the gas bubbles in the tank.

Also applying compression and decompression cycles can help, because pressure variation does not only generate bubbles but increases or decreases the bubble size by respectively decompression or compression. Furthermore, gradual decompression, i.e. releasing pressure in the tank in several steps, can be used to generate bubbles at different depths in the tank in order to achieve a better bubble distribution.

Another way is varying the range of bubble sizes over time. In that way, the period of time during which smaller bubbles are generated will finally deliver the desired range of size at smaller depth. The period of time during which bigger bubbles are generated will finally deliver the desired range of size at larger depth. Although the bubbles grow as they rise, a uniform bubble distribution is obtained in this way.

Also different gasses can be used. Different gasses with different properties allow influencing the final bubble size distribution as well. For example, $N_2$ gas has a lower saturation level in pure wafer than $O_2$ gas. Thus $N_2$ will deliver gas bubbles more easily than $O_2$. If then $N_2$ gas is provided at smaller depth than the $O_2$, a region is created where, as the bubbles rise in the tank, the bubble size is approximately the same for both gasses. Optionally, more than 2 gasses can be used for further optimization.

Further, surfactants can be used to lower the surface tension of the liquid. The gas bubble size will increase then.

When using several transducers with different frequency a multiple frequency field will be created in the tank. In that way, a broader range of bubble sizes can be controlled with respect to cavitation. A megasonic field of 1 MHz affects bigger bubbles than one of 2 MHz. If the megasonic field of 1 MHz is supplied at smaller depth than the 2 MHz field, a broader range of bubble sizes will be affected in the same way, resulting in a broader region of uniform transient cavitation in the tank.

An additional acoustic field can be supplied at larger depth than the depth where the desired range of bubble size has to be achieved. In that way an additional acoustic field with the right specifications can make a particular range of bubbles collapse before entering the region where the final range of size is desired.

Furthermore, a set-up as shown in figure 6 can be used. An additional acoustic field at the bottom of the tank generated by a first set of transducers comprising a plurality of transducers with a same first frequency, is used to create bubbles with a particular range of size. At smaller depth in the tank a second set of transducers comprising a plurality of transducers with a same second frequency is positioned such that the grown bubbles collapse. The first set of transducers and the second set of transducers comprise a same number of transducers. Because of the fixed liquid flow and the fixed distance (d) between a single transducer of the first set of transducers at the bottom and a corresponding single transducer of the second set of transducers at smaller depth, every bubble generated at a single transducer of the first set of transducers at the bottom will grow in the same amount and will collapse by the acoustic field of the corresponding single transducer of the second set of transducers at smaller depth.

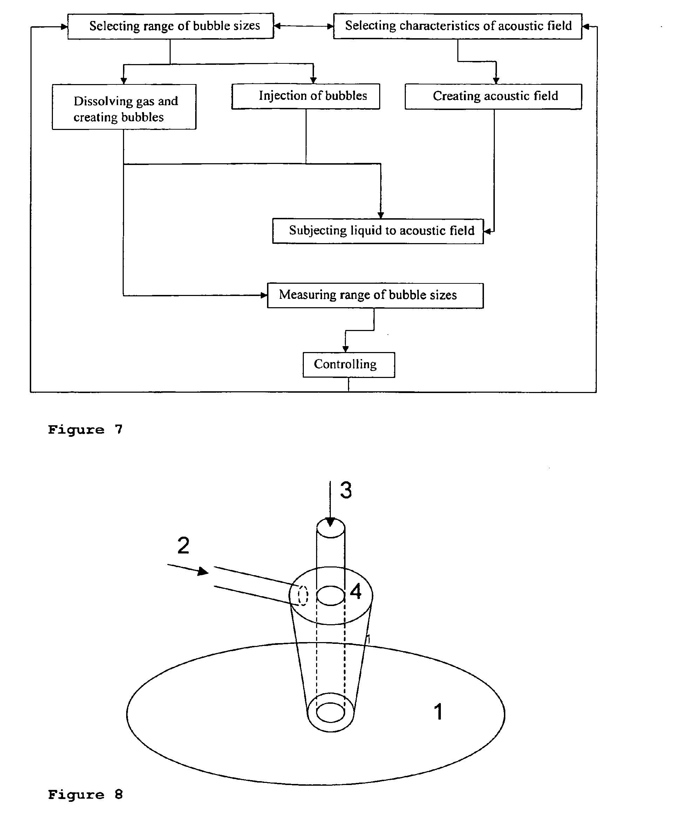

Figure 7 describes the process flow for a particular set-up according to the invention. First the characteristics of the acoustic field are selected, followed by the selection of the range of bubble sizes based on the Rayleigh-Plesset model. This can also be done in the opposite direction. This range of bubble sizes is created by dissolving gas in the liquid and creating bubbles or by immediate injection of gas bubbles in the liquid. If the selected acoustic field is created, the liquid with bubbles in the selected range of bubble sizes is subjected to it. After creating or injecting gas bubbles, the range of bubble sizes is measured by one of the measurement techniques as mentioned before. The results of the measurement are used to control the range of sizes and creation/injection of bubbles and/or the characteristics and creation of the acoustic field. In this way a feed-back loop process is created controlling transient cavitation or bubble collapse in the liquid.

The invention as disclosed in this application can also be used in cases where the acoustic field is not generated in a tank containing the liquid. For example, in semiconductor applications, several batch and single wafer cleaning tools are known wherein a cleaning solution is applied on a rotating wafer 1 and wherein the acoustic field is passed on to the cleaning solution via the wafer 1. Also single wafer cleaning tools are known where the cleaning solution is subjected to an acoustic field during supply by means of a megasonic or ultrasonic spray nozzle. This spray nozzle can be constructed as depicted in figure 8. A deionized water (DIW) supply (2) and a gas+DIW supply (3) are entering the nozzle. The transducer (4) is mounted in the top of the nozzle, surrounding the gas+DIW supply. In this set-up a mixture of DIW and gas, containing gas bubbles with the selected range of bubble size, has to be generated before entering the DIW+gas supply.

Another field of application can be sonochemistry. The method according to this invention can then be used to inject energy from transient cavitation in a controlled way in order to obtain a uniform catalytic effect resulting in a uniform reaction speed.

In ultrasound diagnostics, the bubble size and acoustic field can be optimized to enhance scattering of ultrasound by the bubbles resulting in a better contrast.

Example:

By means of the Young formula, which is based on the Rayleigh-Plesset model, the resonant radius of a bubble for a particular frequency can be calculated:

$$\omega_r^2 = \left( \frac{3\gamma P_0}{\rho R_0^2} \right)$$

If the frequency ($\varphi_r$) = 1.8 MHz and $\omega_r = 2\pi\varphi_r$, the adiabatic constant ($\gamma$) for oxygen = 1.4, the density ($\rho$) for DIW = 1000kg/m$^3$ and the hydrostatic pressure ($P_0$) at 0.25m depth = 103800 Pascal, then the resonant radius is 1.85$\mu$m. This means that in a 1.8MHz acoustic field, even at very low acoustic pressure (e.g. 0.1W/cm$^2$), the bubbles with a radius of 1.85$\mu$m will collapse. At higher acoustic pressure (e.g. 10W/cm$^2$), a broader range of bubbles sizes close to the resonant radius will collapse as well.

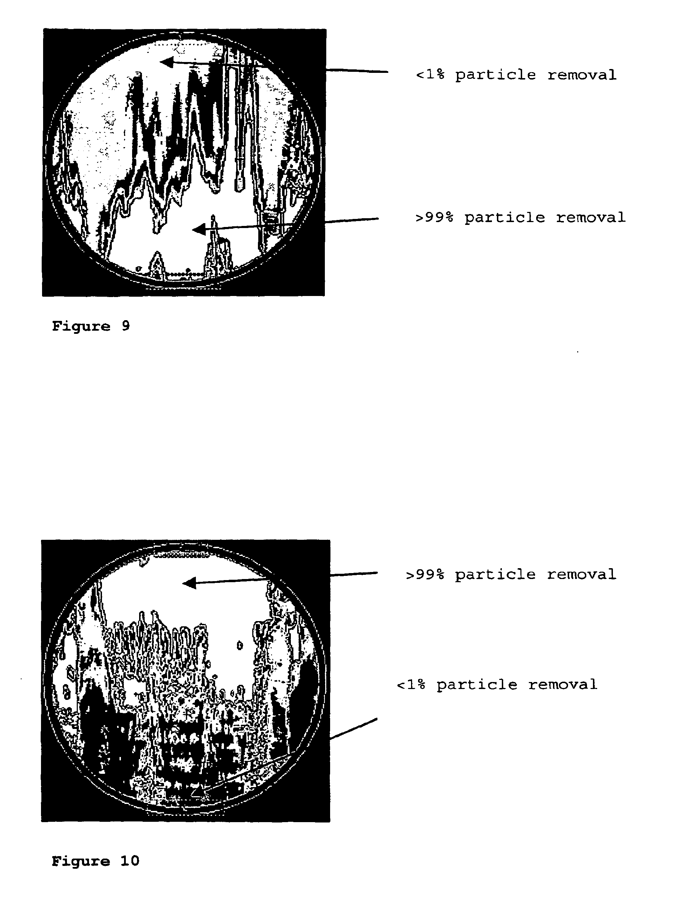

In a particular Techsonic wafer cleaning tank an acoustic field of 1.8 MHz and 5 W/cm$^2$ is generated. The water and gas supply system of the tank, working at 2.5 bar overpressure and containing 18ppm oxygen after passing a Mykrolis Phasor® 2 gasification unit, gives a flow of 8 standard liters per minute (SLM) and fills the tank from the bottom (0.25m depth). The decompression step to 103800 Pascal, occurring at the inlet of the tank, results in a bubble size distribution close to the resonant radius at the bottom of the tank. The results of Particle Removal Efficiency (PRE) for 80nm SiO$_2$ particles on a silicon wafer, measured by the haze channel of the SP1DLS® from KLA-Tencor, are given in figure 9. At the lower part of the wafer PRE is about 99%, where at the top part, PRE is only 1%.

When supplying water at the bottom of the tank containing only 10ppm oxygen, bubbles with a size below the resonant radius are generated. As they rise in the tank, they will grow due to the change in hydrostatic pressure and rectified diffusion till they reach a bubble size distribution close to the resonant radius. In this case the results of PRE are given in figure 10. At the lower part of the wafer PRE is only 1% where at the top part, PRE is about 99%.

Example:

A set of silicon substrates contaminated with 34nm SiO$_2$-particles is used to evaluate the cleaning performance of a single wafer megasonic cleaning tool (Verteq Goldfinger).

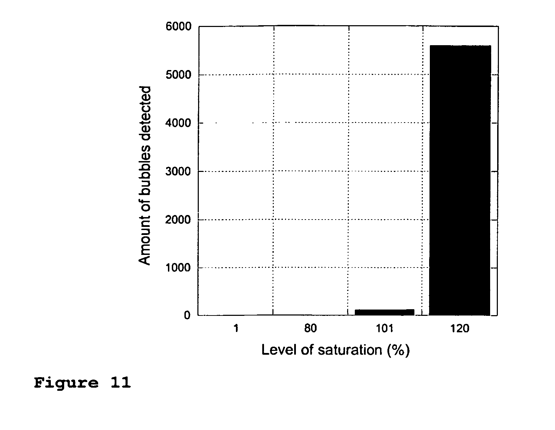

The formation of bubbles, necessary to obtain transient cavitation, is done in the chemical supply system prior to the megasonic field. A backpressure regulator is used to realize a pressure drop, which creates a controlled over-saturation of a specific gas, in this case argon, resulting in a specific bubble distribution. To obtain this bubble distribution it is necessary to gasify the ultra pure water at high pressure ($P_{water}$ = 2.6 bar). The amount of argon added is chosen such that the liquid is under-saturated at the 2.6 bar level (no bubbles were present), but 20% over-saturated after the pressure drop ($P_{water} \sim$ 1bar) . Due to the over-saturation, the excess amount of argon creates a typical bubble distribution in the supply system as shown in figure 11. The generated bubble distribution is monitored by an in-line light scattering tool, designed to measure bubbles larger than 2 micrometer.

The ultra pure water containing the argon bubbles is brought in the megasonic field. The activity of these bubbles in the megasonic field results after 1 minute of processing at 125W input power in a particle removal efficiency of 80% for 34nm SiO$_2$-particles compared to 0% when no gasses were added and 40% when the level of saturation is below 100%.

**Claims**

1. A method for creating transient cavitation comprising the steps of

      - creating gas bubbles having a specific bubble size distribution in a liquid,
      - creating an acoustic field and
      - subjecting the liquid to the acoustic field, **characterized in that** the method furthermore comprises tuning one or both of the bubble size distribution or the characteristics of the acoustic field to each other in accordance with the Rayleigh-Plesset model so as to thereby control transient cavitation in the bubble size distribution.

2. A method according to claim 1, wherein the specific bubble size distribution is selected subsequent to and depending on the selection of the characteristics of the acoustic field.

3. A method according to claim 1, wherein the characteristics of the acoustic field are selected subsequent to and depending on the selection of the specific bubble size distribution.

4. A method according to any of the previous claims, wherein the step of creating gas bubbles having a specific bubble size distribution comprises dissolving gas in the liquid by means of a gasification unit or any means to dissolve gas in the liquid.

5. A method according to any of claims 1 to 3, wherein the step of creating gas bubbles having a specific bubble size distribution comprises injection of gas in the liquid by means of a bubbler system, a capillary, a nozzle, a membrane contactor or any means to inject gas bubbles into the liquid.

6. A method according to any of the previous claims, wherein the step of creating gas bubbles having a specific bubble size distribution comprises a pressure drop or comprises one or multiple compression/decompression cycles.

7. A method according to any of the previous claims, wherein the step of creating gas bubbles having a specific bubble size distribution comprises a raise in temperature or comprises one or multiple cycles of heating/cooling the liquid.

8. A method according to any of the previous claims, wherein the step of creating gas bubbles having a specific bubble size distribution comprises a step of subjecting the liquid to an additional acoustic field.

9. A method according to any of the previous claims, wherein the step of creating gas bubbles having a specific bubble size distribution comprises dissolving or injecting two or more different gasses into the liquid.

10. A method according to any of the previous claims, wherein the step of creating gas bubbles having a specific bubble size distribution comprises adding a surfactant to the liquid.

11. A method according to any of the previous claims, wherein the specific bubble size distribution is varied over time.

12. A method according to any of the previous claims, wherein the acoustic field comprises more than one frequency.

13. A method according to any of the previous claims, further comprising the step of measuring the specific bubble size distribution.

14. A method according to claim 13, further comprising the step of controlling the characteristics of the acoustic field subsequently to and dependent on the measurement.

15. A method according to claim 13, further comprising the step of controlling the specific bubble size distribution subsequently and dependent on the measurement.

16. A method according to any of the above claims, wherein it is used for cleaning semiconductor substrates.

17. An apparatus comprising

   - means for creating gas bubbles having a specific bubble size distribution in a liquid,
   - means for creating an acoustic field and
   - means for subjecting the liquid to the acoustic field, **characterized in that** one or both of the means for creating the gas bubbles or the means for creating the acoustic field are provided with means to tune the bubble size distribution and the characteristics of the acoustic field to each other in accordance with the Rayleigh-Plesset model to thereby control transient cavitation in the bubble size distribution.

18. An apparatus according to claim 17, wherein the means for creating gas bubbles having a specific bubble size distribution comprise a gasification unit or any means to dissolve gas in the liquid.

19. An apparatus according to claim 17, wherein the means for creating gas bubbles having a specific bubble size distribution comprise a valve, a nozzle, a membrane contactor or any means to inject gas bubbles into the liquid.

20. An apparatus according to any of claims 17 to 19, wherein the means for creating gas bubbles having a specific bubble size distribution comprise a heat exchanging system or any means for heating/cooling the liquid.

21. An apparatus according to any of claims 17 to 20, wherein the means for creating gas bubbles having a specific bubble size distribution comprise any means for generating a pressure drop or generating one or multiple compression/decompression cycles.

22. An apparatus according to any of claims 17 to 20, wherein the means for creating gas bubbles having a specific bubble size distribution comprise means to create an additional acoustic field and to subject the liquid to the additional acoustic field.

23. An apparatus according to any of claims 17 to 22, wherein the means for creating gas bubbles having a specific bubble size distribution comprise means for dissolving or injecting two or more different gasses into the liquid.

24. An apparatus according to any of claims 17 to 23, wherein the means for creating gas bubbles having a specific bubble size distribution comprise means for adding a surfactant to the liquid.

25. An apparatus according to any of claims 17 to 24, wherein the means for creating an acoustic field are capable of generating more than one frequency.

26. An apparatus according to any of claims 17 to 25, further comprising a measurement unit for measuring the specific bubble size distribution.

27. An apparatus according to any of claims 17 to 26, further comprising a control unit for controlling one or both of the means for creating gas bubbles having a specific bubble size distribution and the means for creating an acoustic field.

28. An apparatus according to claim 17 to 27, wherein it is used for cleaning semiconductor substrates.


**Patentansprüche**

1. Verfahren zum Erzeugen einer transienten Kavitation, umfassend die folgenden Schritte:

   - Erzeugen von Gasblasen mit einer spezifischen Blasengrößenverteilung in einer Flüssigkeit,
   - Erzeugen eines akustischen Feldes und
   - Aussetzen der Flüssigkeit dem akustischen Feld,

   **dadurch gekennzeichnet, dass** das Verfahren des Weiteren das aufeinander Abstimmen einer oder beider der Blasengrößenverteilung oder der Eigenschaften des akustischen Feldes gemäß dem Rayleigh-Plesset-Modell umfasst, um **dadurch** die transiente Kavitation in der Blasengrößenverteilung zu kontrollieren.

2. Verfahren nach Anspruch 1, wobei die spezifische Blasengrößenverteilung anschließend an und abhängig von der Wahl der Eigenschaften des akustischen Feldes gewählt wird.

3. Verfahren nach Anspruch 1, wobei die Eigenschaften des akustischen Feldes anschließend an und abhängig von der Wahl der spezifischen Blasengrößenverteilung gewählt werden.

4. Verfahren nach einem der vorangehenden Ansprüche, wobei der Schritt des Erzeugens von Gasblasen mit einer spezifischen Blasengrößenverteilung das Auflösen von Gas in der Flüssigkeit mittels einer Vergasungseinheit oder eines beliebigen Mittels zum Auflösen von Gas in der Flüssigkeit umfasst.

5. Verfahren nach einem der Ansprüche 1 bis 3, wobei der Schritt des Erzeugens von Gasblasen mit einer spezifischen Blasengrößenverteilung das Einspritzen von Gas in die Flüssigkeit mittels eines Lufteinperlungssystems, einer Kapillare, einer Düse, eines Membrankontaktors oder eines beliebigen Mittels zum Einspritzen von Gasblasen in die Flüssigkeit umfasst.

6. Verfahren nach einem der vorangehenden Ansprüche, wobei der Schritt des Erzeugens von Gasblasen mit einer spezifischen Blasengrößenverteilung einen Druckabfall umfasst oder einen oder mehrere Kompressions-/Dekompressionszyklen umfasst.

7. Verfahren nach einem der vorangehenden Ansprüche, wobei der Schritt des Erzeugens von Gasblasen mit einer spezifischen Blasengrößenverteilung einen Temperaturanstieg umfasst oder einen oder mehrere Zyklen des Erwärmens/Abkühlens der Flüssigkeit umfasst.

8. Verfahren nach einem der vorangehenden Ansprüche, wobei der Schritt des Erzeugens von Gasblasen mit einer spezifischen Blasengrößenverteilung einen Schritt des Aussetzens der Flüssigkeit einem zusätzlichen akustischen Feld umfasst.

9. Verfahren nach einem der vorangehenden Ansprüche, wobei der Schritt des Erzeugens von Gasblasen mit einer spezifischen Blasengrößenverteilung das Auflösen oder Einspritzen von zwei oder mehreren verschiedenen Gasen in der bzw. in die Flüssigkeit umfasst.

10. Verfahren nach einem der vorangehenden Ansprüche, wobei der Schritt des Erzeugens von Gasblasen mit einer spezifischen Blasengrößenverteilung das Zugeben einer oberflächenaktiven Substanz zu der Flüssigkeit umfasst.

11. Verfahren nach einem der vorangehenden Ansprüche, wobei die spezifische Blasengrößenverteilung im Laufe der Zeit variiert wird.

12. Verfahren nach einem der vorangehenden Ansprüche, wobei das akustische Feld mehr als eine Frequenz umfasst.

13. Verfahren nach einem der vorangehenden Ansprüche, des Weiteren umfassend den Schritt des Messens der spezifischen Blasengrößenverteilung.

14. Verfahren nach Anspruch 13, des Weiteren umfassend den Schritt des Kontrollierens der Eigenschaften des akustischen Feldes anschließend an und abhängig von der Messung.

15. Verfahren nach Anspruch 13, des Weiteren umfassend den Schritt des Kontrollierens der spezifischen Blasengrößenverteilung anschließend an und abhängig von der Messung.

16. Verfahren nach einem der vorangehenden Ansprüche, wobei dieses zum Reinigen von Halbleitersubstraten verwendet wird.

17. Vorrichtung, umfassend:

- ein Mittel zum Erzeugen von Gasblasen mit einer spezifischen Blasengrößenverteilung in einer Flüssigkeit,
- ein Mittel zum Erzeugen eines akustischen Feldes und
- ein Mittel zum Aussetzen der Flüssigkeit dem akustischen Feld,

**dadurch gekennzeichnet, dass** eines oder beide von dem Mittel zum Erzeugen der Gasblasen oder dem Mittel zum Erzeugen des akustischen Feldes mit einem Mittel versehen ist bzw. sind, um die Blasengrößenverteilung und die Eigenschaften des akustischen Feldes gemäß dem Rayleigh-Plesset-Modell aufeinander abzustimmen, um **dadurch** die transiente Kavitation in der Blasengrößenverteilung zu kontrollieren.

18. Vorrichtung nach Anspruch 17, wobei das Mittel zum Erzeugen von Gasblasen mit einer spezifischen Blasengrößenverteilung eine Vergasungseinheit oder ein beliebiges Mittel zum Auflösen von Gas in einer Flüssigkeit umfasst.

19. Vorrichtung nach Anspruch 17, wobei das Mittel zum Erzeugen von Gasblasen mit einer spezifischen Blasengrößenverteilung ein Ventil, eine Düse, einen Membrankontaktor oder ein beliebiges Mittel zum Einspritzen von Gasblasen in die Flüssigkeit umfasst.

20. Vorrichtung nach einem der Ansprüche 17 bis 19, wobei das Mittel zum Erzeugen von Gasblasen mit einer spezifischen Blasengrößenverteilung ein Wärmeaustauschsystem oder ein beliebiges Mittel zum Erwärmen/Kühlen der Flüssigkeit umfasst.

**21.** Vorrichtung nach einem der Ansprüche 17 bis 20, wobei das Mittel zum Erzeugen von Gasblasen mit einer spezifischen Blasengrößenverteilung ein beliebiges Mittel zum Erzeugen eines Druckabfalls oder zum erzeugen eines oder mehrerer Kompressions-/Dekompressionszyklen umfasst.

**22.** Vorrichtung nach einem der Ansprüche 17 bis 20, wobei das Mittel zum Erzeugen von Gasblasen mit einer spezifischen Blasengrößenverteilung ein Mittel zum Erzeugen eines zusätzlichen akustischen Feldes und zum Aussetzen der Flüssigkeit dem zusätzlichen akustischen Feld umfasst.

**23.** Vorrichtung nach einem der Ansprüche 17 bis 22, wobei das Mittel zum Erzeugen von Gasblasen mit einer spezifischen Blasengrößenverteilung ein Mittel zum Auflösen oder Einspritzen von zwei oder mehreren verschiedenen Gasen in der bzw. in die Flüssigkeit umfasst.

**24.** Vorrichtung nach einem der Ansprüche 17 bis 23, wobei das Mittel zum Erzeugen von Gasblasen mit einer spezifischen Blasengrößenverteilung ein Mittel zum Zugeben einer oberflächenaktiven Substanz zu der Flüssigkeit umfasst.

**25.** Vorrichtung nach einem der Ansprüche 17 bis 24, wobei die Mittel zum Erzeugen eines akustischen Feldes imstande sind, mehr als eine Frequenz zu erzeugen.

**26.** Vorrichtung nach einem der Ansprüche 17 bis 25, des Weiteren umfassend eine Messeinheit zum Messen der spezifischen Blasengrößenverteilung.

**27.** Vorrichtung nach einem der Ansprüche 17 bis 26, des Weiteren umfassend eine Steuereinheit zum Steuern eines oder beider von dem Mittel zum Erzeugen von Gasblasen mit einer spezifischen Blasengrößenverteilung und dem Mittel zum Erzeugen eines akustischen Feldes.

**28.** Vorrichtung nach einem der Ansprüche 17 bis 27, wobei diese zum Reinigen von Halbleitersubstraten verwendet wird.

**Revendications**

**1.** Procédé pour créer une cavitation transitoire comprenant les étapes de

- création de bulles de gaz ayant une distribution de taille de bulle spécifique dans un liquide,
- création d'un champ acoustique, et
- soumission du liquide au champ acoustique,

**caractérisé en ce que** le procédé comprend en outre l'accord de l'une ou des deux de la distribution de taille de bulle ou des caractéristiques du champ acoustique l'une à l'autre selon le modèle de Rayleigh-Plesset de façon à commander de ce fait la cavitation transitoire dans la distribution de taille de bulle.

**2.** Procédé selon la revendication 1, dans lequel la distribution de taille de bulle spécifique est sélectionnée suite à et en fonction de la sélection des caractéristiques du champ acoustique.

**3.** Procédé selon la revendication 1, dans lequel les caractéristiques du champ acoustique sont sélectionnées suite à et en fonction de la sélection de la distribution de taille de bulle spécifique.

**4.** Procédé selon l'une quelconque des revendications précédentes, dans lequel l'étape de création de bulles de gaz ayant une distribution de taille de bulle spécifique comprend la dissolution d'un gaz dans le liquide au moyen d'une unité de gazéification ou d'un quelconque moyen pour dissoudre du gaz dans le liquide.

**5.** Procédé selon l'une quelconque des revendications 1 à 3, dans lequel l'étape de création de bulles de gaz ayant une distribution de taille de bulle spécifique comprend l'injection de gaz dans le liquide au moyen d'un système à bulles, d'un capillaire, d'une buse, d'un contacteur à membrane ou d'un quelconque moyen pour injecter des bulles de gaz dans le liquide.

**6.** Procédé selon l'une quelconque des revendications précédentes, dans lequel l'étape de création de bulles de gaz ayant une distribution de taille de bulle spécifique comprend une chute de pression ou comprend un seul ou de multiples cycles de compression / décompression.

7. Procédé selon l'une quelconque des revendications précédentes, dans lequel l'étape de création de bulles de gaz ayant une distribution de taille de bulle spécifique comprend une augmentation de la température ou comprend un seul ou de multiples cycles de chauffage / refroidissement du liquide.

8. Procédé selon l'une quelconque des revendications précédentes, dans lequel l'étape de création de bulles de gaz ayant une distribution de taille de bulle spécifique comprend une étape de soumission du liquide à un champ acoustique supplémentaire.

9. Procédé selon l'une quelconque des revendications précédentes, dans lequel l'étape de création de bulles de gaz ayant une distribution de taille de bulle spécifique comprend la dissolution ou l'injection de deux ou plusieurs gaz différents dans le liquide.

10. Procédé selon l'une quelconque des revendications précédentes, dans lequel l'étape de création de bulles de gaz ayant une distribution de taille de bulle spécifique comprend l'ajout d'un agent de surface au liquide.

11. Procédé selon l'une quelconque des revendications précédentes, dans lequel la distribution de taille de bulle spécifique est modifiée dans le temps.

12. Procédé selon l'une quelconque des revendications précédentes, dans lequel le champ acoustique comprend plus d'une fréquence.

13. Procédé selon l'une quelconque des revendications précédentes, comprenant en outre l'étape de mesure de la distribution de taille de bulle spécifique.

14. Procédé selon la revendication 13, comprenant en outre l'étape de commande des caractéristiques du champ acoustique suite à et en fonction de la mesure.

15. Procédé selon la revendication 13, comprenant en outre l'étape de commande de la distribution de taille de bulle spécifique suite à et en fonction de la mesure.

16. Procédé selon l'une quelconque des revendications précédentes, dans lequel il est utilisé pour le nettoyage de substrats semi-conducteurs.

17. Appareil comprenant

   - un moyen pour créer des bulles de gaz ayant une distribution de taille de bulle spécifique dans un liquide,
   - un moyen pour créer un champ acoustique, et
   - un moyen pour soumettre le liquide au champ acoustique,

   **caractérisé en ce que** l'un ou les deux du moyen pour créer les bulles de gaz ou du moyen pour créer le champ acoustique sont munis d'un moyen pour accorder la distribution de taille de bulle et les caractéristiques du champ acoustique l'une à l'autre selon le modèle de Rayleigh-Plesset pour commander de ce fait la cavitation transitoire dans la distribution de taille de bulle.

18. Appareil selon la revendication 17, dans lequel le moyen de création de bulles de gaz ayant une distribution de taille de bulle spécifique comprend une unité de gazéification ou un quelconque moyen pour dissoudre du gaz dans le liquide.

19. Appareil selon la revendication 17, dans lequel le moyen de création de bulles de gaz ayant une distribution de taille de bulle spécifique comprend une vanne, une buse, un contacteur à membrane ou un quelconque moyen pour injecter des bulles de gaz dans le liquide.

20. Appareil selon l'une quelconque des revendications 17 à 19, dans lequel le moyen de création de bulles de gaz ayant une distribution de taille de bulle spécifique comprend un système d'échange de chaleur ou un quelconque moyen pour chauffer / refroidir le liquide.

21. Appareil selon l'une quelconque des revendications 17 à 20, dans lequel le moyen de création de bulles de gaz ayant une distribution de taille de bulle spécifique comprend un quelconque moyen pour produire une chute de pression ou pour produire un seul ou de multiples cycles de compression / décompression.

22. Appareil selon l'une quelconque des revendications 17 à 20, dans lequel le moyen de création de bulles de gaz ayant une distribution de taille de bulle spécifique comprend un moyen pour créer un champ acoustique supplémentaire et pour soumettre le liquide au champ acoustique supplémentaire.

23. Appareil selon l'une quelconque des revendications 17 à 22, dans lequel le moyen de création de bulles de gaz ayant une distribution de taille de bulle spécifique comprend un moyen pour dissoudre ou injecter deux ou plusieurs gaz différents dans le liquide.

24. Appareil selon l'une quelconque des revendications 17 à 23, dans lequel le moyen de création de bulles de gaz ayant une distribution de taille de bulle spécifique comprend un moyen pour ajouter un agent de surface au liquide.

25. Appareil selon l'une quelconque des revendications 17 à 24, dans lequel le moyen de création d'un champ acoustique est susceptible de produire plus d'une fréquence.

26. Appareil selon l'une quelconque des revendications 17 à 25, comprenant en outre une unité de mesure pour mesurer la distribution de taille de bulle spécifique.

27. Appareil selon l'une quelconque des revendications 17 à 26, comprenant en outre une unité de commande pour commander un ou l'ensemble du moyen de création de bulles de gaz ayant une distribution de taille de bulle spécifique et du moyen de création de champ acoustique.

28. Appareil selon la revendication 17 à 27, dans lequel il est utilisé pour le nettoyage de substrats semi-conducteurs.

Figure 1

Figure 2

**Figure 3**

**Figure 4**

FREQ. vs Pm,Tm

Figure 5

Figure 6

Figure 7

Figure 8

<1% particle removal

>99% particle removal

Figure 9

>99% particle removal

<1% particle removal

Figure 10

Figure 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6048405 A
- US 5931173 A

- DE 4305660 A

**Non-patent literature cited in the description**

- **Neppiras.** Acoustic Cavitation. *Physics Reports,* 1980, vol. 61, 159-251